# EUROPEAN PATENT SPECIFICATION

(11) **EP 3 490 606 B1**
(45) Date of publication and mention of the grant of the patent: **17.01.2024**
(21) Application number: 17749409.3
(22) Date of filing: 25.07.2017
(51) Int. Cl.: A61K 31/585, A61K 45/06, A61K 31/00, A61K 31/7088, A61K 31/713, A61P 19/02

(54) **ANTAGONIST OF MINERALOCORTICOID RECEPTOR FOR THE TREATMENT OF OSTEOARTHRITIS**
MINERALOCORTICOID-RECEPTOR-ANTAGONIST ZUR BEHANDLUNG VON OSTEOARTHRITIS
ANTAGONISTE DES RÉCEPTEURS DES MINÉRALOCORTICOÏDES POUR LE TRAITEMENT DE L'ARTHROSE

(30) Priority: 26.07.2016 EP 16305964
(43) Date of publication of application: 05.06.2019
(73) Proprietor: Institut National de la Santé et de la Recherche Médicale (INSERM), 75013 Paris (FR); Université de Lorraine, 54000 Nancy (FR); Centre National de la Recherche Scientifique CNRS, 75016 Paris (FR)
(72) Inventor: PIZARD, Anne, 54500 Vandoeuvre-Les-Nancy (FR); DENG, Chaohua, 54500 Vandoeuvre-Les-Nancy (FR); KEMPF, Hervé, 54505 Vandoeuvre-Les-Nancy (FR); BIANCHI, Arnaud, 54505 Vandoeuvre-Les-Nancy (FR); MOULIN, David, 54505 Vandoeuvre-Les-Nancy (FR); PRESLE, Nathalie, 54505 Vandoeuvre-Les-Nancy (FR)
(74) Representative: Novagraaf Technologies
(86) International application number: PCT/EP2017/068785
(87) International publication number: WO 2018/019843

(56) References cited:
- US-A1- 2015 174 069
- SYNGLE A ET AL: "Effect of spironolactone on endothelial dysfunction in rheumatoid arthritis.", SCANDINAVIAN JOURNAL OF RHEUMATOLOGY 2009 JAN-FEB, vol. 38, no. 1, January 2009 (2009-01), pages 15-22, XP008182727, ISSN: 1502-7732
- OLIVIER MALAISE ET AL: "Glucocorticoid-induced leucine zipper (GILZ) is involved in glucocorticoid-induced and mineralocorticoid-induced leptin production by osteoarthritis synovial fibroblasts", ARTHRITIS RESEARCH AND THERAPY, vol. 18, no. 1, 4 October 2016 (2016-10-04), pages 219-1, XP055331731, GB ISSN: 1478-6354, DOI: 10.1186/s13075-016-1119-6

## Description

### FIELD OF THE INVENTION:

The present invention relates to an antagonist of mineralocorticoid receptor for use in the treatment of metabolic osteoarthritis in a subject in need thereof, wherein the antagonist of mineralocorticoid receptor is eplerenone.

### BACKGROUND OF THE INVENTION:

Osteoarthritis (OA) is the most common degenerative joint disease among the rheumatic disorders affecting the Western world. This condition is one of the main causes of pain and incapacity in elderly people, becoming a major health problem.

Osteoarthritis is characterized by degeneration of the articular cartilage, remodelling of the subchondral bone and changes in the synovial membrane. It commonly affects the hands, feet, spine, and large extraspinal, weight-bearing joints, such as the hips and knees. The etiology of osteoarthritis is multifactorial involving both mechanical and biochemical factors. Clinical classification includes several phenotypes such as post-traumatic, metabolic, ageing or genetic.

Clinical manifestations of OA are joint pain, stiffness in the morning or after rest, pain at night, limited motion, joint deformity, associated with variable degrees of inflammation of the synovial membrane (synovitis). Joint pain in OA may originate not only from synovitis, but also from stretching of the joint capsule or ligaments, periosteal irritation, trabecular microfractures, intraosseous hypertension or muscle spasms.

Some treatments of osteoarthrosis are available, such as analgesics, nonsteroidal anti-inflammatory drugs, corticosteroids, hyaluronic acid injection, surgery (realigning bones or joint replacement), but these treatments are of high cost and are only focused on controlling and diminishing the pain and inflammation associated with OA disease, and not with controlling, diminishing or eradicating the disease itself. For example, US 2015/174069 A1 discusses anti-inflammatory compounds for the treatment of osteoarthritis.

Thus, there is a need for an improved method to treat osteoarthritis.

### SUMMARY OF THE INVENTION:

The present invention relates to an antagonist of mineralocorticoid receptor for use in the treatment of metabolic osteoarthritis in a subject in need thereof, wherein the antagonist of mineralocorticoid receptor is eplerenone. In particular, the present invention is defined by the claims.

### DETAILED DESCRIPTION OF THE INVENTION:

The inventors showed that accumulated metabolic risk factors, hypertension, obesity, dyslipidemia, insulin resistance (known as metabolic syndrome), lead to severe osteoarthritis articular phenotype. Surprisingly, the inventors showed that preventive and chronic mineralocorticoid receptor antagonist eplerenone treatment can improve the metabolic syndrome related osteoarthritis.

The present invention relates to an antagonist of mineralocorticoid receptor for use in the treatment of metabolic osteoarthritis in a subject in need thereof, wherein the antagonist of mineralocorticoid receptor is eplerenone.

As used herein, the term "subject" denotes a mammal, such as a rodent, a feline, a canine, and a primate. Preferably, a subject according to the invention is a human.

As used herein, the term "osteoarthritis" has its general meaning in the art and refers to a degenerative joint disease with moderate local inflammation occurring chiefly in older humans and animals, which is characterized by degeneration of the articular cartilage, remodelling of the subchondral bone and changes in the synovial membrane. Osteoarthritis can affect any joint of the organism, such as knee, hip, elbow, hands joints, shoulder, back (for example, spinal or cervical), foot joints, ankle, etc. "Metabolic osteoarthritis" is related to an accumulation of metabolic abnormalities or metabolic syndrome.

According to the present invention, osteoarthritis is metabolic osteoarthritis.

As used herein, "treatment" or "treating" is an approach for obtaining beneficial or desired results including clinical results. For purposes of this invention, beneficial or desired clinical results include, but are not limited to, one or more of the following: alleviating one or more symptoms resulting from the disease, diminishing the extent of the disease, stabilizing the disease (e.g., preventing or delaying the worsening of the disease), preventing or delaying the spread of the disease, preventing or delaying the recurrence of the disease, delaying or slowing the progression of the disease, ameliorating the disease state, providing a remission (partial or total) of the disease, decreasing the dose of one or more other medications required to treat the disease, delaying the progression of the disease, increasing the quality of life,
and/or prolonging survival. The term "treatment" encompasses the prophylactic treatment. As used herein, the term "prevent" refers to the reduction in the risk of acquiring or developing a given condition.

As used herein, the term "mineralocorticoid receptor" or "MR" or "aldosterone receptor" has its general meaning in the art and refers to the nuclear receptor subfamily 3, group C, member 2, (NR3C2) that in humans is encoded by the *NR3C2* gene that is located on chromosome 4q31.1-31.2. Mineralocorticoid receptor is a receptor with equal affinity for mineralocorticoids and glucocorticoids.

As used herein, the term "mineralocorticoid receptor antagonist" has its general meaning in the art and refers to any compound that blocks, suppresses, or reduces the biological activity of mineralocorticoid receptor, or to any compound that inhibits mineralocorticoid receptor gene expression. The MR antagonistic of a compound may be determined using various methods as described in J, Souque A, Wurtz JM, Moras D, Rafestin-Oblin ME. Mol Endocrinol. 2000 Aug;14(8):1210-21; Fagart J, Seguin C, Pinon GM, Rafestin-Oblin ME. Mol Pharmacol. 2005 May;67(5):1714-22 or Hellal-Levy C, Fagart J, Souque A, Wurtz JM, Moras D, Rafestin-Oblin ME. Mol Endocrinol. 2000 Aug;14(8):1210-21. Typically, the transfection of the human mineralocorticoid receptor in COS cells together with a luciferase-expressing reporter gene allows to measure its transactivation activity in the presence of a candidate compound. In the present, the mineralocorticoid receptor antagonist is typically selective for the mineralocorticoid receptor as compared with the related receptors such as androgen receptor, estrogen receptors, glucocorticoid receptor, progesterone receptor, thyroid hormone receptors, peroxisome proliferator-activated receptors, retinoic acid receptors, farnesoid x receptor, pregnane x receptor, liver x receptor, vitamin D receptor, retinoid x receptor and the constitutive androstane receptor. By "selective" it is meant that the affinity of the antagonist for the mineralocorticoid receptor is at least 10-fold, typically 25-fold, more typically 100-fold, still typically 500-fold higher than the affinity for the related receptors. MR antagonists constitute a class of pharmacological compounds that are well known by the skilled artisan.

In the present, the antagonist of mineralocorticoid receptor disclosed is a small organic molecule.

For example, the mineralocorticoid receptor antagonists generally are spirolactone-type steroidal compounds. The term "spirolactone-type" is intended to characterize a structure comprising a lactone moiety attached to a steroid nucleus, typically at the steroid "D" ring, through a spiro bond configuration. A subclass of spirolactone-type mineralocorticoid receptor antagonist compounds consists of epoxy-steroidal mineralocorticoid receptor antagonist compounds such as eplerenone. Another subclass of spirolactone-type antagonist compounds consists of non-epoxy-steroidal mineralocorticoid receptor antagonist compounds such as spironolactone.

The epoxy-steroidal mineralocorticoid receptor antagonist compounds disclosed in the present generally have a steroidal nucleus substituted with an epoxy-type moiety. The term "epoxy-type" moiety is intended to embrace any moiety characterized in having an oxygen atom as a bridge between two carbon atoms.

The term "steroidal," as used in the phrase "epoxy-steroidal," denotes a nucleus provided by a cyclopenteno-phenanthrene moiety, having the conventional "A", "B", "C", and "D" rings. The epoxy-type moiety may be attached to the cyclopentenophenanthrene nucleus at any attachable or substitutable positions, that is, fused to one of the rings of the steroidal nucleus or the moiety may be substituted on a ring member of the ring system. The phrase "epoxy-steroidal" is intended to embrace a steroidal nucleus having one or a plurality of epoxy-type moieties attached thereto.
Epoxy-steroidal mineralocorticoid receptor antagonist use in the present invention is Pregn-4-ene-7,21-dicarboxylic acid, 9,11-epoxy-17-hydroxy-3-oxo-,y-lactone, methyl ester, (7α,11α,17β)

A particular benefit of using eplerenone, is the high selectivity of this mineralocorticoid receptor antagonist for the mineralocorticoid receptor. The superior selectivity of eplerenone results in a reduction in side effects that can be caused by mineralocorticoid receptor antagonists that exhibit non-selective binding to related receptors, such as androgen or progesterone receptors.

These epoxy steroids may be prepared by procedures described in Grob et al., U.S. Pat. No. 4,559,332. Additional processes for the preparation of 9, 11-epoxy steroidal compounds and their salts are disclosed in Ng et al., WO97/21720 and Ng et al., WO98/25948.

The compound eplerenone ((Pregn-4-ene-7,21-dicarboxylic acid, 9,11-epoxy-17-hydroxy-3-oxo-,y-lactone, methyl ester, (7α,11α,17β)) (CAS No. 107724-20-9) is also known as epoxymexrenone. Eplerenone is a mineralocorticoid receptor antagonist and has a higher selectivity for mineralocorticoid receptors than does, for example, spironolactone. Selection of eplerenone as the mineralocorticoid receptor antagonist in the present invention is beneficial to reduce certain side-effects such as gynecomastia that occur with use of mineralocorticoid receptor antagonists having less specificity.

According to the present invention, the mineralocorticoid receptor antagonist is eplerenone.

Non-epoxy-steroidal mineralocorticoid receptor antagonists, not being part of the invention, include a family of spirolactone-type compounds defined by Formula I:

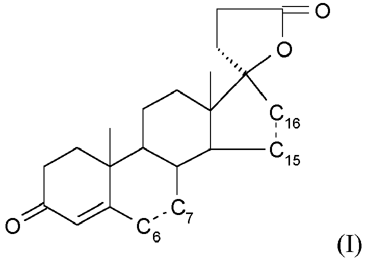

Wherein: 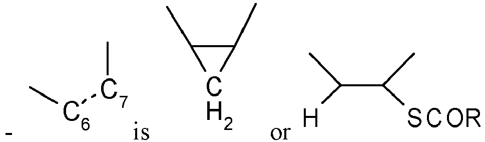 - R is lower alkyl of up to 5 carbon atoms, and

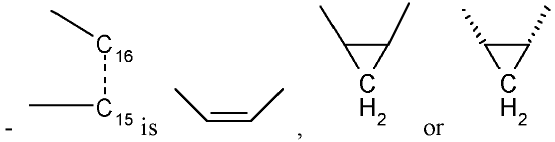

Lower alkyl residues include branched and unbranched groups, for example, methyl, ethyl and n-propyl.

Specific compounds of interest within Formula I are the following:
- 7*α*-acetylthio-3-oxo-4,15-androstadiene-[17(*β*-1')-spiro-5']perhydrofuran-2'-one;
- 3-oxo-7*α*-propionylthio-4,15-androstadiene-[17((*β*-1')-spiro-5']perhydrofuran-2'-one;
- 6*β*,7*β*-methylene-3-oxo4,15-androstadiene-[17((*β*-1')-spiro-5']perhydrofuran-2'-one;
- 15*α*,16*α*-methylene-3-oxo-4,7α-propionylthio-4-androstene[17(*β*-1')-spiro-5']perhydrofuran-2'-one;
- 6*β*,7*β*,15*α,*16*α*-dimethylene-3-oxo-4-androstene[17(*β*-1')-spiro-5']-perhydrofuran-2' -one;
- 7*α*-acetylthio-15*β*,16*β*-Methylene-3-oxo-4-androstene-[17(*β*-1')-spiro-5' ]perhydrofuran-2' -one;
- 15*β*,16*β*-methylene-3-oxo-7*β*-propionylthio-4-androstene-[17(*β*-1')-spiro-5']perhydrofuran-2'-one; and
- 6*β*,7*β*,15*β*,16*β*-dimethylene-3-oxo-4-androstene-[17(*β*-1')-spiro-5']perhydrofuran-2' -one.

Methods to make compounds of Formula I are described in U.S. Pat. No. 4,129,564 to Wiechart et al. issued on 12 Dec. 1978.

Another family of non-epoxy-steroidal compounds, not being part of the invention, is defined by Formula II: 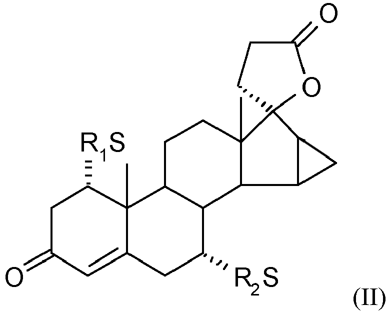 wherein R1 is C1-3-alkyl or C1-3 acyl and R2 is H or C1-3-alkyl.

Specific compounds not being part of the invention, within Formula II are the following:
- 1α-acetylthio-15β,16β-methylene-7α-methylthio-3-oxo-17α-pregn-4-ene-21,17-carbolactone; and
- 15β,16β-methylene-1α,7α-dimethylthio-3-oxo-17α-pregn-4-ene-21,17-carbolactone.

Methods to make the compounds of Formula II are described in U.S. Pat. No. 4,789,668 to Nickisch et al., which issued 6 Dec. 1988.

Yet another family of non-epoxy-steroidal compounds not being part of the invention, is defined by a structure of Formula III: 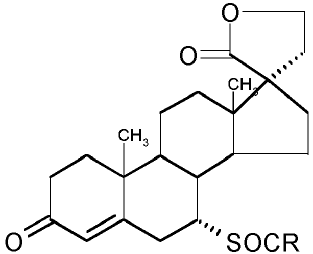 or 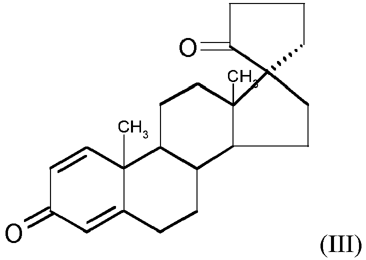 wherein R is lower alkyl, examples of which include lower alkyl groups of methyl, ethyl, propyl and butyl. Specific compounds of interest include:
- 3β,21-dihydroxy-17α-pregna-5,15-diene-17-carboxylic acid γ-lactone;
- 3β,21-dihydroxy-17α-pregna-5,15-diene-17-carboxylic acid γ-lactone 3-acetate;
- 3β,21-dihydroxy-17α-pregn-5-ene-17-carboxylic acid γ-lactone;
- 3β,21-dihydroxy-17α-pregn-5-ene-17-carboxylic acid γ-lactone 3-acetate;
- 21-hydroxy-3-oxo-17α-pregn-4-ene-17-carboxylic acid γ-lactone;
- 21-hydroxy-3-oxo-17α-pregna-4,6-diene-17-carboxylic acid γ-lactone;
- 21-hydroxy-3-oxo-17α-pregna-1,4-diene-17-carboxylic acid γ-lactone;
- 7α-acylthio-21-hydroxy-3-oxo-17α-pregn-4-ene-17-carboxylic acid γ-lactone; and
- 7α-acetylthio-21-hydroxy-3-oxo-17α-pregn-4-ene-17-carboxylic acid γ-lactone.

Methods to make the compounds of Formula III are described in U.S. Pat. No. 3,257,390 to Patchett, which issued 21 Jun. 1966.

Still another family of non-epoxy-steroidal compounds not being part of the invention, is represented by Formula IV: 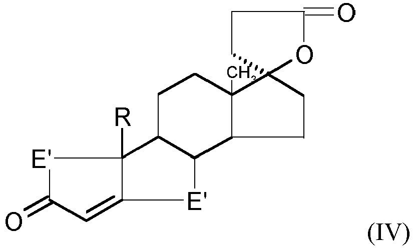 wherein E' is selected from the group consisting of ethylene, vinylene and (lower alkanoyl)thioethylene radicals, E" is selected from the group consisting of ethylene, vinylene, (lower alkanoyl)thioethylene and (lower alkanoyl)thiopropylene radicals; R is a methyl radical except when E' and E" are ethylene and (lower alkanoyl) thioethylene radicals, respectively, in which case R is selected from the group consisting of hydrogen and methyl radicals; and the selection of E' and E" is such that at least one (lower alkanoyl)thio radical is present.

One family of non-epoxy-steroidal compounds within Formula IV is represented by Formula V:

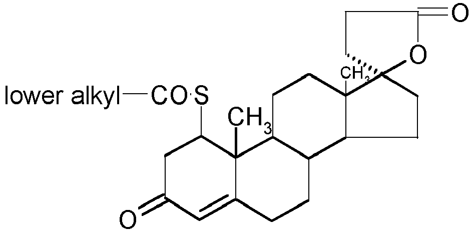

Another compound of Formula V is 1-acetylthio-17α-(2-carboxyethyl)-17β-hydroxy-androst-4-en-3-one lactone.

Another family of non-epoxy-steroidal compounds within Formula IV not being part of the invention, is represented by Formula VI:

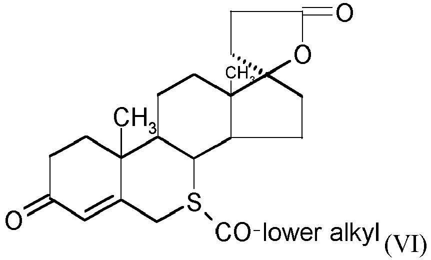

Exemplary compounds within Formula VI include the following:
- 7α-acetylthio-17α-(2-carboxyethyl)-17β-hydroxy-androst-4-en-3-one lactone;
- 7β-acetylthio-17α-(2-carboxyethyl)-17β-hydroxy-androst-4-en-3-one lactone;
- 1α,7α-diacetylthio-17α-(2-carboxyethyl)-17β-hydroxy-androsta-4,6-dien-3-one lactone;
- 7α-acetylthio-17αe-(2-carboxyethyl)-17β-hydroxy-androsta-1,4-dien-3-one lactone;
- 7α-acetylthio-17α-(2-carboxyethyl)-17β-hydroxy-19-norandrost-4-en-3-one lactone; and
- 7α-acetylthio-17α-(2-carboxyethyl)-17β-hydroxy-6α-methylandrost-4-en-3-one lactone.

In Formulae IV-VI, the term "alkyl" is intended to embrace linear and branched alkyl radicals containing one to about eight carbons. The term "(lower alkanoyl)thio" embraces radicals of the formula lower alkyl

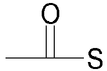

The compound spironolactone (17-hydroxy-7α-mercapto-3-oxo-17α-pregn-4-ene-21-carboxylic acid γ-lactone acetate) have the following structure:

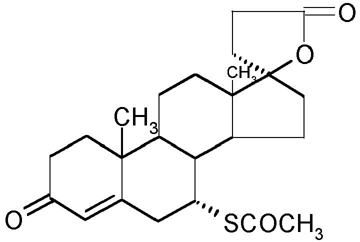

Methods to make compounds of Formulae IV-VI are described in U.S. Pat. No. 3,013,012 to Cella et al., which issued 12 Dec. 1961. Spironolactone is sold by G. D. Searle & Co., Skokie, Ill., under the trademark "ALDACTONE", in tablet dosage form at doses of 25 mg, 50 mg and 100 mg per tablet.

Another family of steroidal mineralocorticoid receptor antagonists not being part of the invention, is exemplified by drospirenone, (6R-(6*α,*7*α,*8*β*,9*α,*10*β*,13*β*,14*α*, 15*α*,16*α*,17*β*))-1, 3' , 4' , 6, 7, 8, 9, 10, 11, 12, 13, 14, 15, 16, 20, 21-hexadecahydro-10, 13-dimethylspiro [17H-dicyclopropa(6,7:15,16)cyclopenta(a)phenanthrene-17,2' (5' H)-furan)-3,5' (2H)-dione, CAS registration number 67392-87-4. Methods to make and use drospirenone are described in patent GB 1550568 1979, priority DE 2652761 1976.

Crystalline forms that are easily handled, reproducible in form, easily prepared, stable, and which are non-hygroscopic have been identified for the mineralocorticoid receptor antagonist eplerenone. These include Form H, Form L, various crystalline solvates and amorphous eplerenone. These forms, methods to make these forms, and use of these forms in preparing compositions and medicaments, are disclosed in Barton et al., WO 01/41535 and Barton et al., WO 01/42272.

Mineralocorticoid receptor antagonists not being part of the invention, may also be non-steroidal. For example, classes of non-steroidal MR antagonists have just begun to emerge over the past few years (Meyers, Marvin J1; Hu, Xiao Expert Opinion on Therapeutic Patents, Volume 17, Number 1, January 2007 , pp. 17-23(7) and Piotrowski DW. Mineralocorticoid Receptor Antagonists for the Treatment of Hypertension and Diabetic NephropathyJ. Med. Chem. 2012, 55, 7957-7966). For instance, dihydropyrymidines have been shown to display MR antagonism (Activation of Mineralocorticoid Receptors by Exogenous Glucocorticoids and the Development of Cardiovascular Inflammatory Responses in Adrenalectomized Rats. Young MJ, Morgan J, Brolin K, Fuller PJ, Funder JW. Endocrinology. 2010 Apr 21). Furthermore, Arhancet el al. disclose other class of non-steroidal MR antagonists (Arhancet GB, Woodard SS, Dietz JD, Garland DJ, Wagner GM, Iyanar K, Collins JT, Blinn JR, Numann RE, Hu X, Huang HC. Stereochemical Requirements for the Mineralocorticoid Receptor Antagonist Activity of Dihydropyridines. J Med Chem. 2010 Apr 21). Other exemplary non-steroidal mineralocorticoid receptor antagonists include but are not limited to those described in US 20090163472 WO2004052847, WO 2008053300 WO2008104306, WO2007025604, WO201264631, WO2008126831, WO2012008435, WO2010104721, WO200985584, WO200978934, WO2008118319, WO200917190, WO200789034, WO2012022121, WO2012022120, WO2011141848 and WO200777961.

Disclosed mineralocorticoid receptor antagonist is selected from the group consisting of:

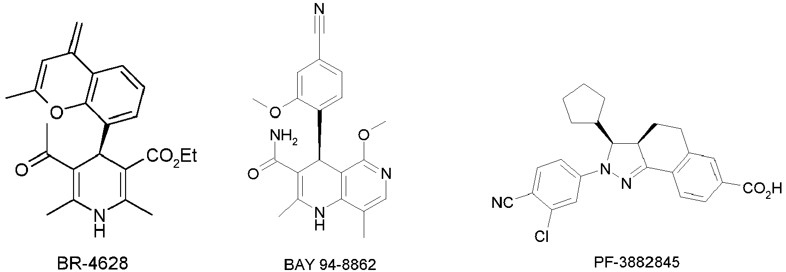

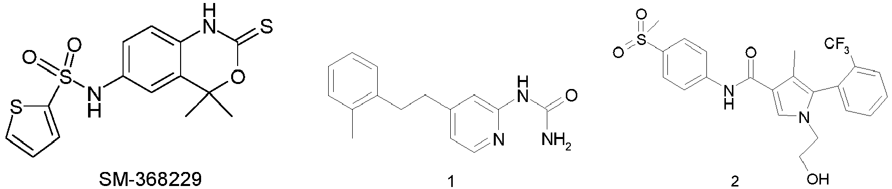

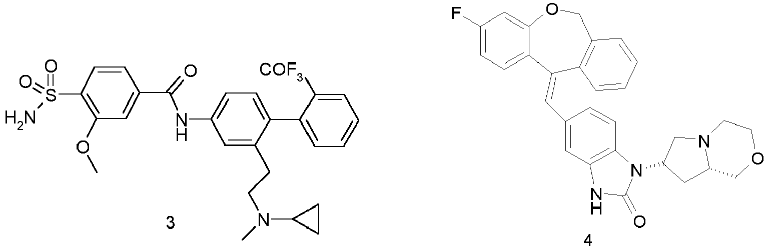

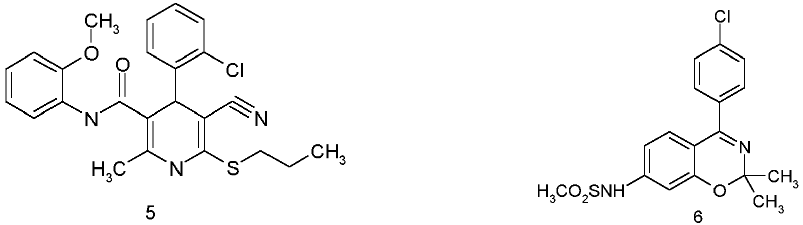

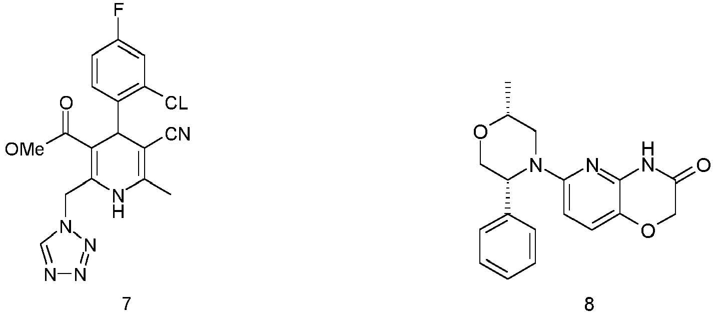

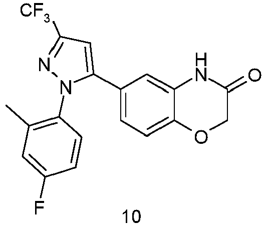

In the present, disclosed mineralocorticoid receptor antagonist is an inhibitor of expression. In particular, disclosed mineralocorticoid receptor antagonist is an inhibitor of mineralocorticoid receptor antagonist gene expression.

Said disclosed inhibitor of gene expression is a siRNA, an antisense oligonucleotide or a ribozyme.

In the present, the disclosed mineralocorticoid receptor antagonist is anti-sense oligonucleotides.

Anti-sense oligonucleotides, including anti-sense RNA molecules and anti-sense DNA molecules, would act to directly block the translation of target gene mRNA by binding thereto and thus preventing protein translation or increasing mRNA degradation, thus decreasing the level of mineralocorticoid receptor subunit thereof, and thus activity, in a cell. For example, antisense oligonucleotides complementary to unique regions of the mRNA transcript sequence encoding mineralocorticoid receptor can be synthesized, e.g., by conventional phosphodiester techniques. Methods for using antisense techniques for specifically inhibiting gene expression of genes whose sequence is known are well known in the art (e.g. see U.S. Pat. Nos. 6,566,135; 6,566,131; 6,365,354; 6,410,323; 6,107,091; 6,046,321; and 5,981,732).

In the present, disclosed mineralocorticoid receptor antagonist is a small inhibitory RNA.

Small inhibitory RNAs (siRNAs) can function as inhibitors of mineralocorticoid receptor gene expression. Mineralocorticoid receptor gene expression can be reduced by contacting a subject or cell with a small double stranded RNA (dsRNA), or a vector or construct causing the production of a small double stranded RNA, such that mineralocorticoid receptor gene expression is specifically inhibited (i.e. RNA interference or RNAi). Methods for selecting an appropriate dsRNA or dsRNA-encoding vector are well known in the art for genes whose sequence is known (e.g. see for example Tuschl, T. et al. (1999); Elbashir, S. M. et al. (2001); Hannon, GJ. (2002); McManus, MT. et al. (2002); Brummelkamp, TR. et al. (2002); U.S. Pat. Nos. 6,573,099 and 6,506,559; and International Patent Publication Nos. WO 01/36646, WO 99/32619, and WO 01/68836).

In the present, disclosed mineralocorticoid receptor antagonist is a ribozyme.

Ribozymes can also function as inhibitors of mineralocorticoid receptor gene expression. Ribozymes are enzymatic RNA molecules capable of catalyzing the specific cleavage of RNA. The mechanism of ribozyme action involves sequence specific hybridization of the ribozyme molecule to complementary target RNA, followed by endonucleolytic cleavage. Engineered hairpin or hammerhead motif ribozyme molecules that specifically and efficiently catalyze endonucleolytic cleavage of mineralocorticoid receptor mRNA sequences are disclosed. Specific ribozyme cleavage sites within any potential RNA target are initially identified by scanning the target molecule for ribozyme cleavage sites, which typically include the following sequences, GUA, GUU, and GUC. Once identified, short RNA sequences of between about 15 and 20 ribonucleotides corresponding to the region of the target gene containing the cleavage site can be evaluated for predicted structural features, such as secondary structure, that can render the oligonucleotide sequence unsuitable. The suitability of candidate targets can also be evaluated by testing their accessibility to hybridization with complementary oligonucleotides, using, e.g., ribonuclease protection assays.

Both antisense oligonucleotides and ribozymes useful as inhibitors of mineralocorticoid receptor gene expression can be prepared by known methods. These include techniques for chemical synthesis such as, e.g., by solid phase phosphoramadite chemical synthesis. Alternatively, anti-sense RNA molecules can be generated by in vitro or in vivo transcription of DNA sequences encoding the RNA molecule. Such DNA sequences can be incorporated into a wide variety of vectors that incorporate suitable RNA polymerase promoters such as the T7 or SP6 polymerase promoters. Various modifications to the oligonucleotides can be introduced as a means of increasing intracellular stability and half-life. Possible modifications include but are not limited to the addition of flanking sequences of ribonucleotides or deoxyribonucleotides to the 5' and/or 3' ends of the molecule, or the use of phosphorothioate or 2'-O-methyl rather than phosphodiesterase linkages within the oligonucleotide backbone.

Antisense oligonucleotides siRNAs and ribozymes may be delivered in vivo alone or in association with a vector. In its broadest sense, a "vector" is any vehicle capable of facilitating the transfer of the antisense oligonucleotide siRNA or ribozyme nucleic acid to the cells and preferably cells expressing mineralocorticoid receptor. Preferably, the vector transports the nucleic acid to cells with reduced degradation relative to the extent of degradation that would result in the absence of the vector. In general, the vectors include, but are not limited to, plasmids, phagemids, viruses, other vehicles derived from viral or bacterial sources that have been manipulated by the insertion or incorporation of the antisense oligonucleotide siRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rouse sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus.

Some viral vectors are based on non-cytopathic eukaryotic viruses in which non-essential genes have been replaced with the gene of interest. Non-cytopathic viruses include retroviruses (e.g., lentivirus), the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in Kriegler, 1990 and in Murry, 1991.

Viruses for certain applications are the adeno-viruses and adeno-associated viruses, which are double-stranded DNA viruses. The adeno-associated virus can be engineered to be replication deficient and is capable of infecting a wide range of cell types and species. It further has advantages such as, heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well known to those of skill in the art. See e.g. Sambrook et al., 1989. In the last few years, plasmid vectors have been used as DNA vaccines for delivering antigen-encoding genes to cells in vivo. They are particularly advantageous for this because they do not have the same safety concerns as with many of the viral vectors. These plasmids, however, having a promoter compatible with the host cell, can express a peptide from a gene operatively encoded within the plasmid. These plasmids are well known to those of ordinary skill in the art. Additionally, plasmids may be custom designed using restriction enzymes and ligation reactions to remove and add specific fragments of DNA. Plasmids may be delivered by a variety of parenteral, mucosal and topical routes. For example, the DNA plasmid can be injected by intramuscular, eye, intradermal, subcutaneous, intra-articular or other routes. It may also be administered by intranasal sprays or drops, rectal suppository and orally. It may also be administered into the epidermis or a mucosal surface using a gene-gun. The plasmids may be given in an aqueous solution, dried onto gold particles or in association with another DNA delivery system including but not limited to liposomes, dendrimers, cochleate and microencapsulation.

Is disclosed in the present, the antisense oligonucleotide, siRNA, shRNA or ribozyme nucleic acid sequence under the control of a heterologous regulatory region, e.g., a heterologous promoter.

Is disclosed in the present, the mineralocorticoid receptor antagonist of the invention administered to the subject with a therapeutically effective amount.

The terms "administer" or "administration" refer to the act of injecting or otherwise physically delivering a substance as it exists outside the body (e.g., mineralocorticoid receptor antagonist of the present invention) into the subject, such as by mucosal, intradermal, intravenous, subcutaneous, intramuscular, intra-articular delivery and/or any other method of physical delivery described herein or known in the art. When a disease, or a symptom thereof, is being treated, administration of the substance typically occurs after the onset of the disease or symptoms thereof. When a disease or symptoms thereof, are being prevented, administration of the substance typically occurs before the onset of the disease or symptoms thereof.

By a "therapeutically effective amount" is meant a sufficient amount of mineralocorticoid receptor antagonist for use in a method for the treatment of osteoarthritis at a reasonable benefit/risk ratio applicable to any medical treatment. It will be understood that the total daily usage of the compounds and compositions of the present invention will be decided by the attending physician within the scope of sound medical judgment. The specific therapeutically effective dose level for any particular subject will depend upon a variety of factors including the severity of the OA, the age, body weight, general health, sex and diet of the subject; the time of administration, route of administration, and rate of excretion of the specific compound employed; the duration of the treatment; and like factors well known in the medical arts. For example, it is well known within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Typically, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the subject to be treated. A medicament typically contains from about 0.01 mg to about 500 mg of the active ingredient, typically from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

The compositions are formulated for parenteral, transdermal, oral, rectal, subcutaneous, sublingual, topical or intranasal administration.

Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

In one embodiment, the compositions are formulated for parenteral administration. The pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

In the present, the compositions according to the invention are preferably formulated for intra-articular administration.

In the present, the compositions according to the invention are preferably formulated for intravenous administration.

In the present, the compositions according to the invention are also formulated for oral administration.

Typically the active ingredient of the present invention (i.e. the mineralocorticoid receptor antagonist) is combined with pharmaceutically acceptable excipients, and optionally sustained-release matrices, such as biodegradable polymers, to form pharmaceutical compositions.

The term "pharmaceutically" or "pharmaceutically acceptable" refers to molecular entities and compositions that do not produce an adverse, allergic or other untoward reaction when administered to a mammal, especially a human, as appropriate.

A pharmaceutically acceptable carrier or excipient refers to a non-toxic solid, semisolid or liquid filler, diluent, encapsulating material or formulation auxiliary of any type. The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin. In the pharmaceutical compositions of the present invention, the active ingredients of the invention can be administered in a unit administration form, as a mixture with conventional pharmaceutical supports.

In some embodiments, the mineralocorticoid receptor antagonist of the present invention is administered to the subject in combination with an active ingredient.

In some embodiments, the mineralocorticoid receptor antagonist of the present invention is administered to the subject in combination with a standard treatment. For instance, standard treatment of osteoarthritis is analgesics, nonsteroidal anti-inflammatory drugs, corticosteroids, hyaluronic acid injection, surgery, physical activity, weight management, etc.

The invention will be further illustrated by the following figures and examples. However, these examples and figures should not be interpreted in any way as limiting the scope of the present invention.

### FIGURES:

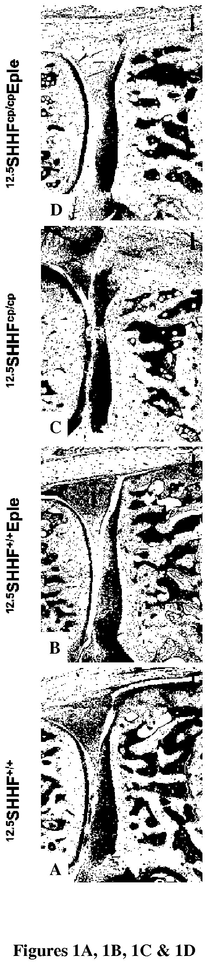

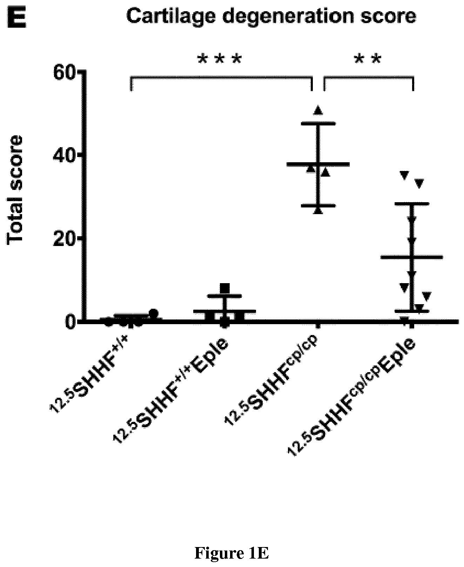

**Figure 1****: Chronic treatment of mineralocorticoid receptor antagonist eplerenone prevents the degradation of joint cartilage.** Representative photographs of the knee joint of the ^{12.5}SHHF^{+/+} rats (A), ^{12.5}SHHF^{+/+}Eple rats (B), the ^{12.5}SHHF^{cp/cp} rats (C) and the ^{12.5}SHHF^{cp/cp} Eple rats (D). Sections were stained with toluidine blue. Scale bar: 100µm. OARSI scoring of the joint cartilage degradation (E) in the ^{12.5}SHHF^{+/+} rats (n=4), ^{12.5}SHHF^{+/+}Eple rats (n=4), the ^{12.5}SHHF^{cp/cp} rats (n=4) and the ^{12.5}SHHF^{cp/cp} Eple rats (n=9). Values represent mean±SD. One-way ANOVA with Bonferroni's correction was used for statistical analysis.

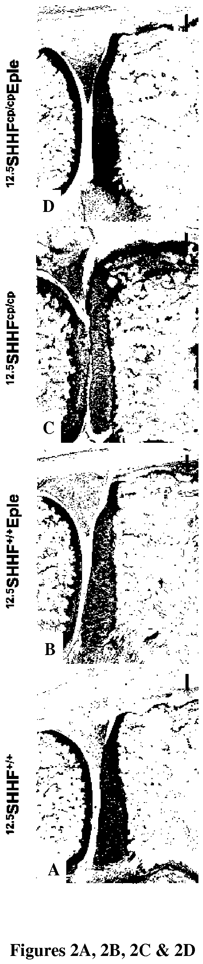

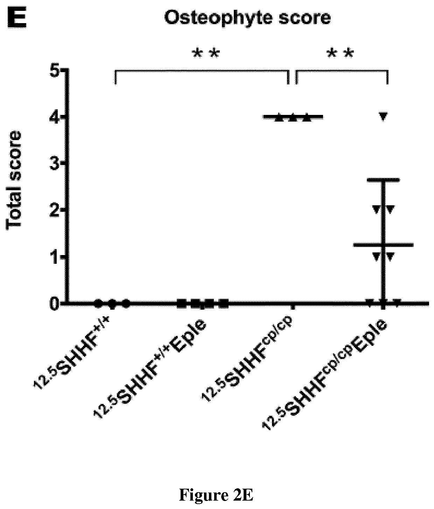

**Figure 2****: Chronic treatment of mineralocorticoid receptor antagonist eplerenone reduces osteophyte formation.** Representative photographs of the knee joint of the ^{12.5}SHHF^{+/+} rats (A), ^{12.5}SHHF^{+/+}Eple rats (B), the ^{12.5}SHHF^{cp/cp} rats (C) and the ^{12.5}SHHF^{cp/cp} Eple rats (D). Sections were immunostained with an antibody against collagen II. Scale bar: 100µm. OARSI scoring of osteophyte in the ^{12.5}SHHF^{+/+} rats (n=4), ^{12.5}SHHF^{+/+}Eple rats (n=4), the ^{12.5}SHHF^{cp/cp} rats (n=4) and the ^{12.5}SHHF^{cp/cp}Eple rats (n=9). Values represent mean±SD. One-way ANOVA with Bonferroni's correction was used for statistical analysis.

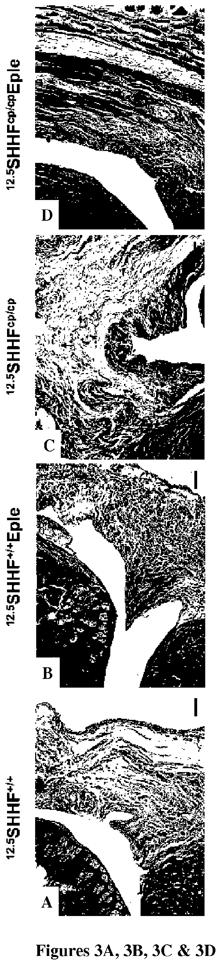

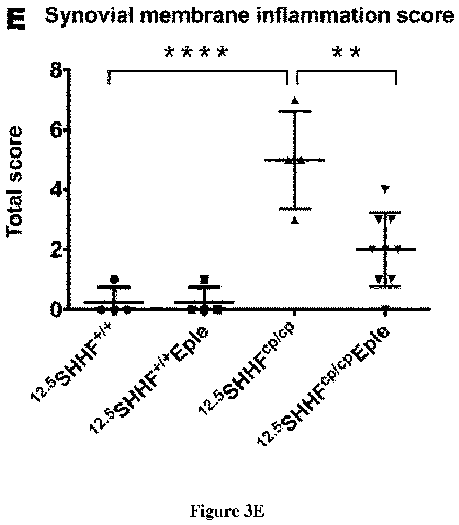

**Figure 3****: Chronic treatment of mineralocorticoid receptor antagonist eplerenone reduces the joint inflammation.** Representative photographs of the knee joint of the ^{12.5}SHHF^{+/+} rats (A), ^{12.5}SHHF^{+/+}Eple rats (B), the ^{12.5}SHHF^{cp/cp} rats (C) and the ^{12.5}SHHF^{cp/cp} Eple rats (D). Sections were stained with Hematoxylin & Eosin. Scale bar: 20µm. OARSI scoring of the joint inflammation (E) in the ^{12.5}SHHF^{+/+} rats (n=4), ^{12.5}SHHF^{+/+}Eple rats (n=4), the ^{12.5}SHHF^{cp/cp} rats (n=4) and the ^{12.5}SHHF^{cp/cp}Eple rats (n=9). Values represent mean±SD. One-way ANOVA with Bonferroni's correction was used for statistical analysis.

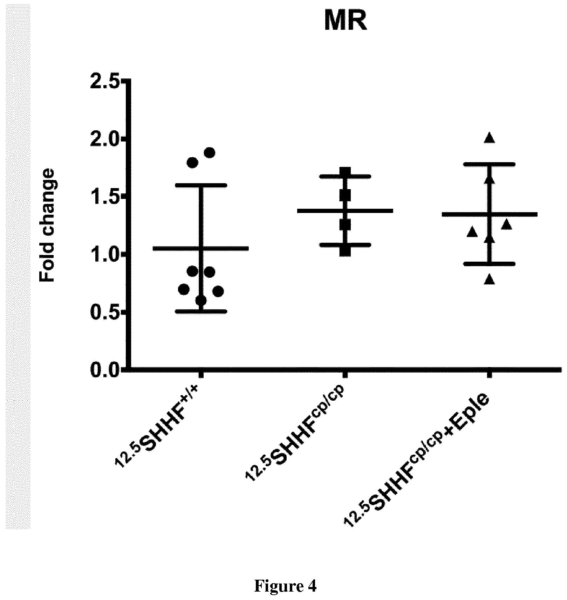

**Figure 4****: Mineralocorticoid receptor gene expression in femoral head cartilage** from ^{12.5}SHHF^{+/+} rats (n=7), ^{12.5}SHHF^{cp,cp} rats (n=4) and ^{12.5}SHHF^{cp/cp}Eple rats (n=6). Values represent mean±SD. Unpaired two-tailed t test was used for statistical analysis.

### EXAMPLE:

### Material & Methods

In this study were used lean SHHF^{+/+} (spontaneously hypertensive heart failure) rats as controls and obese SHHF^{cp/cp} rats, which have been characterized as a good model for metabolic syndrome (MetS). To ease the description of the different experimental groups, the time (expressed in month) when the observations were performed is displayed as an exponent before the strain SHHF (as an example: ^{1.5}SHHF referring to observations done at 1.5 months of age), the genotype is indicated by an exponent after SHHF (as SHHF^{+/+} or SHHF^{cp/cp}) and the treatment status with eplerenone "Eple" follows. The absence of eple means that the considered rats are part of a control placebo group.

Animals of both lean SHHF^{+/+} and MetS SHHF^{cp/cp} genotypes were divided randomly into two groups. Group I (n=4 for SHHF^{+/+} and n=4 for SHHF^{cp/cp}) was given placebo and group II (n=4 for SHHF^{+/+} and n=9 for SHHF^{cp/cp}) was given 100 mg.kg-1.day-1 of the selective MRA eplerenone (pure active molecule provided by Pfizer) in drinking water from 1.5 months to 12.5 months of age. The knee joints of 1.5- and 12.5- month-old specimens of each group (untreated SHHF^{+/+} and SHHF^{cp/cp} and their treated counterparts) were fixed in 4% paraformaldehyde, decalcified in EDTA-phosphate buffered solution and then embedded in paraffin. Five µm thick adjacent sections were stained with Toluidine Blue, Hematoxylin and Eosin or submitted to collagen II immunohistochemistry in order to investigate cartilage destruction, osteophyte formation and lesions of the synovial tissue, respectively. The corresponding scores following OARSI guidelines were done blindly by independent researchers.

For MR gene expression study, femoral head cartilage was harvested from ^{12.5}SHHF^{+/+}, ^{12.5}SHHF^{cp/cp} and ^{12.5}SHHF^{cp/cp}Eple rats. Total RNA was extracted. Reverse transcription and PCR using MR specific primers were performed to detect the MR transcript in this tissue.

### Results

At 1.5 months of age, histological observations of joints of both lean ^{1.5}SHHF^{+/+} and ^{1.5}SHHF^{cp/cp} rats showed no abnormal articular phenotype (not shown). ^{1.5}SHHF^{+/+} and ^{1.5}SHHF^{cp/cp} were then undistinguishable.

### In placebo groups of rats at 12.5 months:

- The knee joints of the ^{12.5}SHHF^{+/+} rats displayed normal phenotype. If any, extremely faint and focal OA-like lesions were characterized by some surface irregularities with no or mild proteoglycans loss in articular cartilage (Fig1A), absence of collagen II-positive osteophytes (Fig2A) and very moderate fibrosis of the synovial membrane (Fig3A).
- In contrast to age-matched ^{12.5}SHHF^{+/+}, the joints of MetS ^{12.5}SHHF^{cp/cp} rats exhibit hallmarks of grade II to grade III OA. Extreme OA lesions were characterized by marked fibrillations from the surface to the middle layer of the cartilage and moderate to severe loss of proteoglycans through the entire thickness of the cartilage in some areas (Fig.1C). Furthermore, OA scoring statistically revealed that the articular cartilage was significantly deteriorated in ^{12.5}SHHF^{cp/cp} compared to age-matched ^{12.5}SHHF^{+/+} (Fig1E). Interestingly, the medial plateau exibits more severe cartilage destruction that is very comparable to the human features of OA. Additional analysis of adjacent sections revealed that those alterations of the cartilage are associated with presence of osteophyte and changes in the synovial tissue. Thus, important osteophytes (compare Fig2B vs. Fig2A) as well as fibrosis, inflammation and cellular infiltration are observed in the ^{12.5}SHHF^{cp/cp} rats (compare Fig3C vs. Fig3A). OARSI score for soteophyte (Fig2E) and synovial membrane inflammation (Fig3E) confirmed these observations These findings clearly suggest that the MetS condition found in ^{12.5}SHHF^{cp/cp} rats is responsible for the development of a spontaneous severe OA phenotype.

In eplerenone groups of rats at 12.5 months:
- The knee joints of the ^{12.5}SHHF^{+/+}eple rats displayed normal phenotype just like their untreated placebo counterparts ^{12.5}SHHF (Fig.1B&E, 2B&E, 3B&E),.
- In striking contrast, compared to the severe OA lesions observed in placebo ^{12.5}SHHF^{cp/cp} rats, the knee joints of MetS ^{12.5}SHHF^{cp/cp}Eple rats treated from 1.5 months onward display phenotypes very similar to those of their ^{12.5}SHHF^{+/+} counterparts. The beneficial effect of eplerenone treatment on ^{12.5}SHHF^{cp/cp}Eple knee joints includes markedly reduced fibrillations and loss of proteoglycans (compare Fig1D vs. Fig1C), alleviated osteophytes (compare Fig2D vs. Fig2C), as well as attenuated inflammation and fibrosis of the synovial tissue (compare Fig3D vs. Fig3C) as compared to ^{12.5}SHHF^{cp/cp} untreated rats. Thus, 11-month eplerenone treatment drastically decreases the cartilage alterations (Fig1E), significantly hampers osteophyte formation (Fig2E) and markedly reduces the level of inflammatory and fibrotic events (Fig3E) that were typical of non-treated SHHF^{cp/cp} rats.

Altogether, this demontrates that chronic MRA treatment is able to notably prevent the development of severe OA lesions in the OA-prone SHHF^{cp/cp} rodent model.

While MRA are known to reduce inflammation and fibrosis at the systemic level, our results showing MR gene expression in the joint cartilage of the rat (Fig4) strongly suggest a yet to be confirmed potential local role of the MR in OA development and consequently of MRA in OA treatment

### Conclusion

These results are of utmost importance in two ways:
1. We have uncovered SHHF^{cp/cp} rats as the first spontaneous metabolic-associated OA model in rat. This has obvious implications for advancing our understanding of « metabolic » OA as SHHF^{cp/cp} strain may become very instrumental in testing/defining new therapeutics in this context.
2. Moreover, we have evidenced that the chronic antagonisation of MR (by eplerenone) could positively impede or at least slow down the development of OA-lesions in the joints of individuals with metabolic disorders. As a safe and already largely used medication in patients with cardiovascular diseases, MR antagonism may thus constitute the first identified therapeutic strategy effective for OA that potentially may avoid/delay surgical knee replacement. Interestingly, through amelioration of OA-features and consequent relief of associated pain, preventive MRA could increase mobility and quality of life of OA patients with MetS and therefore indirectly participate to the decrease of cardiovascular risks in MetS patients by preserving physical activity.

### REFERENCES:

Throughout this application, various references describe the state of the art to which this invention pertains.

## Claims

1. An antagonist of mineralocorticoid receptor for use in the treatment of metabolic osteoarthritis in a subject in need thereof, wherein the antagonist of mineralocorticoid receptor is eplerenone.

2. The antagonist of mineralocorticoid receptor for use according to claims 1 wherein the antagonist of mineralocorticoid receptor is administered to the subject in combination with a standard treatment.

## Patentansprüche

1. Mineralocorticoidrezeptor-Antagonist zur Verwendung bei der Behandlung von metabolischer Osteoarthritis in einem Patienten, der dessen bedarf, wobei der Mineralocorticoidrezeptor-Antagonist Eplerenon ist.

2. Antagonist des Mineralocorticoidrezeptors zur Verwendung gemäß Anspruch 1, wobei der Antagonist des Mineralocorticoidrezeptors dem Patienten in Kombination mit einer Standardbehandlung verabreicht wird.

## Revendications

1. Antagoniste du récepteur minéralocorticoïde pour utilisation dans le traitement de l'arthrose métabolique chez un sujet qui en a besoin, dans lequel l'antagoniste du récepteur minéralocorticoïde est l'éplérénone .

2. Antagoniste du récepteur minéralocorticoïde utilisé selon la revendication 1, dans lequel l'antagoniste du récepteur minéralocorticoïde est administré au sujet en association avec un traitement standard.
